# FASCICULE DE BREVET EUROPEEN

(11) **EP 2 751 251 B1**
(45) Date de publication et mention de la délivrance du brevet: **07.03.2018**
(21) Numéro de dépôt: 12762339.5
(22) Date de dépôt: 30.08.2012
(51) Int. Cl.: C12M 1/00, C12M 1/26, C12M 1/22, C12Q 1/24

(54) **PROCEDES D'ENSEMENCEMENT DE MILIEUX DE CULTURE SUR BOITES DE PETRI PAR FREQUENCES VIBRATOIRES**
VERFAHREN ZUM EINIMPFEN VON KULTURMEDIEN AUF PETRISCHALEN MITTELS SCHWINGUNGSFREQUENZEN
METHODS FOR INOCULATING CULTURE MEDIA ON PETRI DISHES BY MEANS OF VIBRATION FREQUENCIES

(30) Priorité: 30.08.2011 FR 1157619
(43) Date de publication de la demande: 09.07.2014
(73) Titulaire: Biomérieux, 69280 Marcy l'Étoile (FR)
(72) Inventeur: COLIN, Bruno, F-69280 Marcy L'Etoile (FR); MONTET, Marie-Pierre, 69200 Vénissieux (FR)
(86) Numéro de dépôt international: PCT/FR2012/051956
(87) Numéro de publication internationale: WO 2013/030510

(56) Documents cités:
- WO-A1-2006/087398
- DE-A1- 19 631 997
- DE-A1-102010 006 473
- GB-A- 1 278 531
- JP-A- 2008 131 928
- US-A- 4 344 712

## Description

Le domaine technique de la présente invention est celui de la microbiologie. Plus particulièrement, la présente invention concerne un procédé d'ensemencement, avec un échantillon, de milieux de culture contenus dans des boites Petri, par fréquences vibratoires.

Dans les domaines du diagnostic clinique et du contrôle microbiologique industriel agroalimentaire, pharmaceutique ou cosmétique, les milieux de culture gélosés en boite de Petri constituent depuis plusieurs décennies un outil indispensable à la détection et l'identification des microorganismes, éventuellement pathogènes.

L'ensemencement de tels milieux de culture se fait de manière classique, manuellement, à l'aide d'un outil d'ensemencement qui est une oese en plastique jetable, une anse de platine qu'il est nécessaire de chauffer à fins de stérilisation entre deux utilisations. L'ensemencement peut également être réaliser à l'aide d'une pipette à usage unique.

L'ensemencement peut également se faire de manière automatique à l'aide de systèmes développés et commercialisés à cette fin. De tels systèmes ont en fait pour fonction d'automatiser l'ensemencement de milieux de culture en boites de Petri, à l'aide de parties mécaniques robotisées. Un tel système est par exemple commercialisé par la demanderesse sous la dénomination PREVI® Isola.

Que ce soit par le biais de techniques manuelles ou automatisées, l'ensemencement des milieux de culture sur boites de Petri nécessite une ouverture prolongée de cette dernière pour permettre le processus d'ensemencement. Ce dernier consiste généralement en un isolement ou un dénombrement, à savoir que l'outil d'ensemencement qui porte l'échantillon susceptible de contenir des microorganismes et qui est mis en contact avec le milieu de culture, est déplacé sur ce dernier afin de déposer l'échantillon, et donc les microorganismes, sur le milieu de culture. Ainsi, cette ouverture prolongée peut entrainer des risques de contamination du milieu de culture par des microorganismes autres que ceux présents dans l'échantillon, venant de l'environnement extérieur. Par ailleurs, il est également susceptible de se produire l'effet inverse, à savoir que l'ouverture prolongée de la boite lors de l'ensemencement est susceptible d'entrainer une contamination de l'environnement extérieur par le ou les microorganismes présents dans l'échantillon.

Dans le document WO-A-2006/087398, il est décrit un dispositif permettant de collecter un échantillon liquide susceptible de contenir des microorganismes dans un premier compartiment haut et de réaliser une ensemencement d'un milieu de culture en boite de Petri, sous forme de spray, la boite de Petri étant placé dans un deuxième compartiment bas. La pulvérisation est obtenue par passage de l'échantillon au travers de micro-buses, lesdites micro-buses étant ouvertes par le biais d'une pression exercée par un moyen de pression.

Si un tel dispositif peut s'avérer pratique dans certains cas précis, il présente comme inconvénient majeur de nécessiter plusieurs étapes de manipulation pour notamment mettre en place l'échantillon et la boite de Petri. Ceci est difficilement envisageable dans le cadre de l'activité soutenue d'un laboratoire de microbiologie dans lequel sont manipulés plusieurs dizaines de prélèvements par jour.

Le document DE 10 2010 006 473 décrit une boite de Petri dont le couvercle présente un trou traversant fermé par un septum. A ce titre, la boite de Petri décrite dans ce document permet l'ensemencement du milieu de culture contenu dans ladite boite, sans ouvrir le couvercle et ainsi, limiter les risques de contamination.

L'ensemencement de la boite de Petri est réalisé à l'aide d'un dispositif d'ensemencement accessoire qui est positionné à l'intérieur de la boite et solidaire du couvercle de ladite boite. Ce dispositif est constitué d'une lame radiale terminée par une levre qui entre en contact avec le milieu de culture lors de l'ensemencement, réalisé par rotation du couvercle sur le fond de boite.

L'invention décrite dans le document DE 10 2010 006 473 présente comme principal inconvénient de complexifier la boite de Petri, alors que cette dernière est à la base un produit d'une simplicité extrême, ce qui en fait son succès. Par ailleurs, l'utilisation d'un système d'ensemencement tel que décrit ne permet pas de réaliser des étalements à des fins d'isolement, performants.

Le document DE 196 31 997 décrit une boite de Petri dont le couvercle présente une buse de pulvérisation et un système de compensation de pression. Une pulvérisation du fluide à introduire dans la boite, est réalisée sous forme de spray à l'aide d'un dispositif sous pression venant se connecter sur la buse de pulvérisation. L'invention décrite dans le document DE 196 31 997 présente comme inconvénient principal d'être relativement complexe par rapport à une boite de Petri traditionnelle qui se veut extrêmement simple. Ceci génère nécessairement un surcoût important lors de la fabrication d'un tel produit.

Au regard de l'analyse de l'existant, il apparaît donc comme particulièrement intéressant de pouvoir disposer d'une méthode d'ensemencement limitant les risques de contamination tout en étant simple à mettre en oeuvre, facilement adaptable dans le cadre d'un laboratoire de microbiologie traditionnel et ne générant pas de modifications trop importantes de la boite de Petri qui est l'outil de base du laboratoire de microbiologie.

La présente invention se propose de résoudre les problèmes techniques abordés ci-dessus en proposant un procédé d'ensemencement d'au moins un milieu de culture gélosé, contenu dans une boite de Petri, avec au moins un échantillon susceptible de contenir des microorganismes, ledit procédé comportant les étapes consistant à :
a) Fournir au moins un milieu de culture gélosé contenu dans une boite de Pétri ;
b) Fournir l'échantillon susceptible de contenir des microorganismes sous forme liquide ;
c) Effectuer au moins un dépôt de l'échantillon, sous forme liquide, sur le couvercle de la boite de Petri de sorte qu'un transfert dudit échantillon du couvercle vers le milieu de culture gélosé, soit possible ;
d) Pulvériser l'échantillon, initialement déposé sur le couvercle, sur le milieu de culture gélosé, par mise en vibration de la boite de Petri.

Selon un mode de réalisation particulier de l'invention, le dépôt de l'échantillon est effectué sur la face intérieure du couvercle de la boite de Petri.

Avantageusement, le dépôt de l'échantillon sur la face intérieure du couvercle, est effectuée au travers dudit couvercle. Ledit dépôt de l'échantillon liquide peut être notamment réalisé au travers d'un septum. Alternativement, le dépôt de l'échantillon liquide est réalisé au travers d'un trou traversant réalisé dans le couvercle. Ledit trou peut être réalisé par perçage, avec l'outil de dépôt de l'échantillon liquide.

Selon un mode de réalisation alternatif de l'invention, la boite de Petri est ouverte afin d'effectuer le dépôt d'échantillon sur la face intérieure du couvercle de ladite boite de Petri.

Selon un autre mode de réalisation alternatif de l'invention, le dépôt est effectué dans au moins une cavité, ménagée dans le couvercle. La cavité est ouverte sur la face extérieure du couvercle et présente un orifice au niveau de la face intérieure dudit couvercle.

Avantageusement, durant l'étape de pulvérisation, la vibration de la boite de Petri est obtenue par mise en contact de cette dernière avec un moyen de vibration. Plus avantageusement encore, c'est le couvercle de la boite de Petri qui est mis en contact avec un moyen de vibration.

Les buts et avantages des procédés selon la présente invention seront mieux compris à la lumière de l'exemple nullement limitatif qui suit, en référence au dessin, dans lequel :
La figure 1A représente une vue en perspective d'une boite de Petri selon un premier mode de réalisation.
La figure 1B représente un grossissement de la boite de Petri représentée sur la figure 1A.
La figure 2A représente une coupe transversale de la boite de Petri selon le premier mode de réalisation.
La figure 2B représente une coupe transversale de la boite de Petri selon le premier mode de réalisation, lors de l'étape de dépôt d'une goutte d'un échantillon à analyser.
La figure 2C représente une coupe transversale de la boite de Petri selon le premier mode de réalisation, lors de l'étape de pulvérisation de l'échantillon liquide, par mise en vibration de la boite de Petri.
La figure 2D représente une coupe transversale de la boite de Petri selon le premier mode de réalisation, une fois l'échantillon liquide réparti sur le milieu de culture sous forme de gouttelettes et la boite de Petri incubée pendant un temps nécessaire au développement des microorganismes.
La figure 3A représente une coupe transversale de la boite de Petri selon un deuxième mode de réalisation.
La figure 3B représente une coupe transversale de la boite de Petri selon le deuxième mode de réalisation, lors de l'étape de dépôt d'une goutte d'un échantillon à analyser.
La figure 3C représente une coupe transversale de la boite de Petri selon le deuxième mode de réalisation, lors de l'étape de pulvérisation de l'échantillon liquide, par mise en vibration de la boite de Petri.
La figure 3D représente une coupe transversale de la boite de Petri selon le deuxième mode de réalisation, une fois l'échantillon liquide réparti sur le milieu de culture sous forme de gouttelettes et la boite de Petri incubée pendant un temps nécessaire au développement des microorganismes.
La figure 4A représente une coupe transversale de la boite de Petri selon un troisième mode de réalisation.
La figure 4B représente une coupe transversale de la boite de Petri selon le troisième mode de réalisation, lors de l'étape de dépôt d'une goutte d'un échantillon à analyser.
La figure 4C représente une coupe transversale de la boite de Petri selon le troisième mode de réalisation, lors de l'étape de pulvérisation de l'échantillon liquide, par mise en vibration de la boite de Petri.
La figure 4D représente une coupe transversale de la boite de Petri selon le troisième mode de réalisation, une fois l'échantillon liquide réparti sur le milieu de culture sous forme de gouttelettes et la boite de Petri incubée pendant un temps nécessaire au développement des microorganismes.

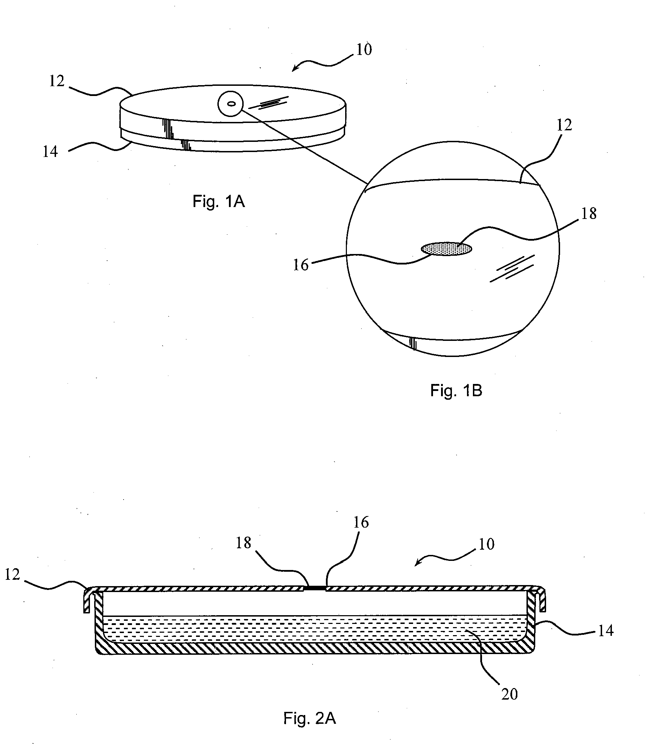

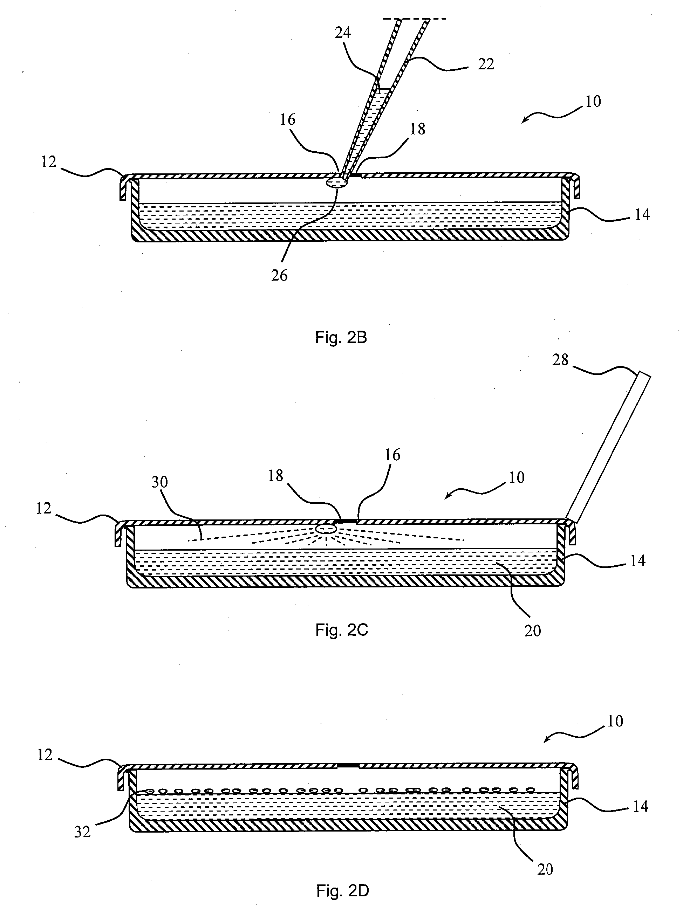

Un premier mode de réalisation 10 du procédé selon l'invention est représenté en perspective sur les figures 1A et 1B et en coupe transversale sur la figure 2A. La boite de Petri 10 est constituée d'un couvercle 12 et d'un fond 14. Ces deux éléments sont classiquement en matériau polymère transparent, tel que du polystyrène. De façon nullement limitative, la boite de Petri 10 est ici représentée sous sa forme traditionnelle, à savoir cylindrique à base ronde. Le couvercle 12 de la boite de Petri 10 présente en son centre, un trou traversant 16. Ce trou traversant 16 est ici obturé avec une membrane 18. Cette membrane doit être dans un matériau permettant de la percer. Dans un mode préférentiel de réalisation, cette membrane 16 est une membrane élastique de type septum, qui peut être percée à l'aide d'un outil approprié et qui dispose par ailleurs de la capacité de se refermer. De tel septums sont bien connus et largement utilisés dans le domaine médical. Comme on peut le voir sur la figure 2A, la boite de Petri comporte par ailleurs en son sein une couche de milieu de culture gélosé 20, susceptible d'être ensemencée par le biais d'une méthode d'ensemencement selon l'invention, qui va être décrite ci-après, en lien avec les figures 2B à 2D.

Sur la figure 2B, est représentée l'étape de dépôt d'une goutte d'échantillon liquide. Plus précisément, cette étape consiste en premier à aspirer à l'aide d'un dispositif d'aspiration/refoulement 22 tel qu'une pipette, une fraction d'un échantillon 24 à analyser, sous forme liquide. Un tel échantillon peut être réellement un échantillon liquide, tel que :
- liquide corporel, et notamment urine, sang, liquide articulaire ;
- échantillon liquide d'origine alimentaire tel qu'une boisson ;
- échantillon environnemental, tel qu'un prélèvement d'eau ;
- échantillon pharmaceutique ou cosmétique.

L'échantillon liquide 24 peut également être un milieu d'enrichissement auquel a été mélangé un échantillon primaire. Un tel échantillon primaire peut être un échantillon liquide tel que décrit supra mais également un échantillon solide, tel qu'un échantillon alimentaire.

Enfin, l'échantillon liquide 24 peut également être une suspension de microorganismes réalisée à partir d'un isolat. Un tel isolat peut par exemple être constitué de colonies bactériennes.

Le dispositif d'aspiration/refoulement 22, rempli avec la fraction d'échantillon 24 à analyser est utilisé pour percer la membrane 18 de façon à permettre à l'embout dudit dispositif d'aspiration/refoulement 22 de traverser le couvercle 12 au travers du trou traversant 16. L'embout du dispositif d'aspiration/refoulement 22 est alors mis en contact avec la face interne du couvercle 12 de façon à déposer une goutte 26 de l'échantillon 24 sur ladite face interne.

Une fois la goutte 26 déposée, le dispositif d'aspiration/refoulement 22 est retiré. La membrane élastique 18 se referme, limitant ainsi les risques de contamination à l'intérieur de la boite de Petri 10. Il peut tout à fait être envisagé de faire plusieurs dépôts sous forme de gouttes 26 sur la face interne du couvercle 12.

L'étape suivante représentée sur la figure 2C, consiste à générer des vibrations du couvercle 12 afin d'entraîner la pulvérisation de l'échantillon 24 à partir de la goutte 26 déposée sur la face interne du couvercle 12. A cette fin, il est fait usage d'un moyen de vibration 28, qui est mis en contact avec le couvercle 12. Un tel moyen de vibration est par exemple une roue crantée entrainée par un moteur et venant au contact de la boite de Petri, préférentiellement du couvercle de ladite boite. Tout autre moyen permettant de générer des vibrations et bien connu de l'homme du métier peut également être utilisé.

Lorsque le moyen de vibration 28 est mis en oeuvre, il génère des vibrations au niveau du couvercle 12. La fréquence de ces vibrations est préférentiellement comprise entre 800 et 4500 hertz (Hz).Ces vibrations génèrent alors une pulvérisation par projection de microgouttes 30 d'échantillon à partir de la goutte 26, en particulier en direction du milieu de culture gélosé. Cette pulvérisation génère une répartition aléatoire à la surface du milieu de culture gélosé, de microgouttes d'échantillon susceptibles de contenir des microorganismes tels que des bactéries. Le résultat obtenu est ainsi sensiblement équivalent à un ensemencement traditionnel d'un milieu de culture par technique d'isolement.

Après incubation de la boite de Petri dans une étuve durant une période comprise entre 12 et 72h, on observe à la surface du milieu de culture gélosé 20, l'apparition de colonies bactériennes 32 isolées les unes des autres, tel que représenté sur la figure 2D.

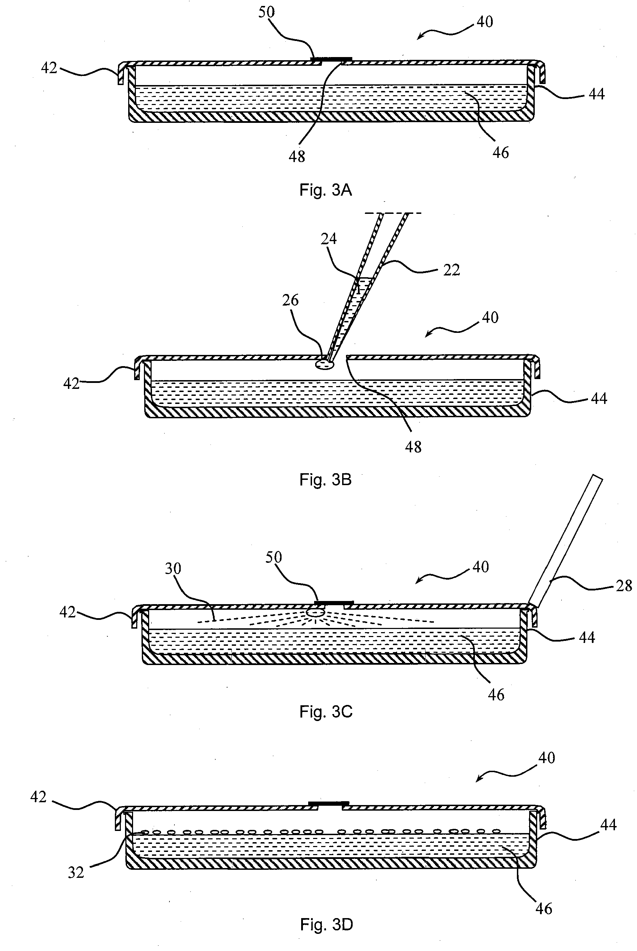

Un deuxième mode de réalisation du procédé d'ensemencement est représenté sur les figures 3A à 3D.

Sur la figure 3A, on observe une boite de Petri 40, constituée d'un couvercle 42 et d'un fond 44. Le couvercle 42 vient se positionner sur le fond 44. A l'intérieur de cette boite de Petri se trouve un milieu de culture gélosé 46.

Le couvercle 42 de la boite de Petri 40 présente en son centre, un trou traversant 48. Ce trou traversant 48 est ici obturé par une languette 50, évitant ainsi toute communication entre l'intérieur de la boite de Petri et l'environnement extérieur. Cette languette 50 peut être constituée de tout matériau adapté à la fonction qu'elle revêt. Elle peut par exemple être à base de papier ou de matériau polymère. Elle peut être monocouche ou multicouches. Elle dispose avantageusement sur la face en contact avec le couvercle de la boite de Petri d'une couche d'un matériau adhésif, dont le pouvoir adhésif doit permettre de décoller ladite languette mais également de la recoller ; éventuellement à plusieurs reprises. Il est par ailleurs important que les dimensions de la languette 50 soient bien supérieures aux dimensions du trou traversant 48, afin de limiter au maximum les échanges entre l'extérieur et l'intérieur de la boite de Petri.

Sur la figure 3B, est représentée l'étape de dépôt d'une goutte d'échantillon liquide. Plus précisément, cette étape consiste en premier à aspirer à l'aide d'un dispositif d'aspiration/refoulement 22, une fraction d'un échantillon 24 à analyser, sous forme liquide.

Afin de permettre le dépôt d'une ou plusieurs gouttes de l'échantillon 24 sur la face interne du couvercle 42, il est procédé au décollement de la languette 50 (non représenté sur la figure 3B). Le décollement de la languette 50 peut être partiel ou total. En effet, il peut être envisagé de décoller la languette 50 partiellement de manière à libérer le trou traversant 48 ou de désolidariser cette dernière complètement du couvercle 42. C'est cette alternative qui est représentée sur la figure 3B. Chacune de ces alternatives présente des avantages et des inconvénients. Un décollement partiel présente l'avantage de permettre un recollement plus facile. Il présente néanmoins comme inconvénient d'augmenter les risques de contamination de la languette 50 avec l'échantillon 24 par le biais de l'embout du dispositif d'aspiration/refoulement 22, lors l'introduction de ce dernier au travers du trou traversant 48. Un décollement complet de la languette 50 présente comme principal avantage d'empêcher tout risque de contamination de cette dernière. Par contre, elle nécessite de placer la languette 50 dans un endroit sûr le temps nécessaire au dépôt de l'échantillon 24 dans la boite de Petri 40 puis de repositionner ladite languette 50 de manière optimale sur le couvercle afin d'assurer une obturation efficace du trou traversant 48.

Une fois le trou traversant 48 rendu accessible, le dispositif d'aspiration/refoulement 22, rempli avec la fraction d'échantillon 24 à analyser est positionné de façon à permettre à l'embout dudit dispositif d'aspiration/refoulement 22 de traverser le couvercle 12 au travers du trou traversant 48. L'embout du dispositif d'aspiration/refoulement 22 est alors mis en contact avec la face interne du couvercle 12 de façon à déposer au moins une goutte 26 de l'échantillon 24 sur ladite face interne.

Une fois la ou les gouttes 26 déposées, le dispositif d'aspiration/refoulement 22 est retiré. La languette 50 est alors repositionnée, limitant ainsi les risques de contamination à l'intérieur de la boite de Petri 40. Ceci est présenté sur la figure 3C. Il est alors possible de générer des vibrations du couvercle 12 afin d'entraîner la pulvérisation de l'échantillon 24 à partir de la goutte 26 déposée sur la face interne du couvercle 12. A cette fin, il est fait usage d'un moyen de vibration 28, mis en contact avec le couvercle 12, comme déjà explicité supra, en lien avec la figure 2C. Ces vibrations génèrent alors une pulvérisation par projection de microgouttes 30 d'échantillon à partir de la goutte 26, en particulier en direction du milieu de culture gélosé. Cette pulvérisation génère une répartition aléatoire à la surface du milieu de culture gélosé, de microgouttes d'échantillon susceptibles de contenir des microorganismes tels que des bactéries. Le résultat obtenu est ainsi sensiblement identique à un ensemencement traditionnel d'un milieu de culture par technique d'isolement.

Après incubation de la boite de Petri dans une étuve durant une période comprise entre 12 et 72h, on observe à la surface du milieu de culture gélosé 46, l'apparition de colonies bactériennes 32 isolées les unes des autres, tel que représenté sur la figure 3D.

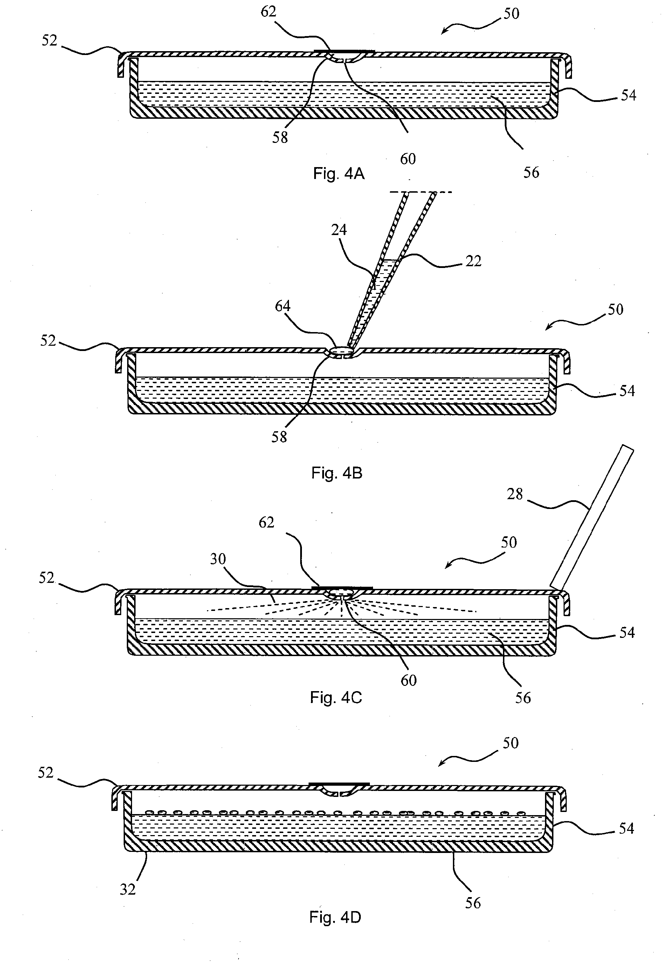

Un troisième mode de réalisation du procédé d'ensemencement est représenté en lien avec les figures 4A à 4D. La boite de Petri 50, selon ce troisième mode de réalisation, est constituée d'un couvercle 52 et d'un fond 54. Le couvercle 52 vient se positionner sur le fond 54. A l'intérieur de cette boite de Petri se trouve un milieu de culture gélosé 56.

Le couvercle 52 de la boite de Petri 50 présente en son centre, une cavité 58 ménagée dans ledit couvercle 52 et destinée à recevoir un dépôt d'échantillon liquide à analyser. Cette cavité 58 comporte en son fond un trou traversant 60, de sorte qu'elle est en communication fluidique avec l'intérieur de la boite de Petri 50. La cavité 58 est obturée dans sa partie supérieure par une languette 62, évitant ainsi d'une part, la contamination de ladite cavité et d'autre part, toute communication entre l'intérieur de la boite de Petri et l'environnement extérieur. Cette languette 62 est identique ou similaire à la languette 50 décrite *supra* et son mode d'utilisation également.

Ainsi, sur la figure 4B, est représentée l'étape de dépôt d'une goutte d'échantillon liquide à l'intérieur de la cavité . Plus précisément, cette étape consiste en premier à aspirer à l'aide d'un dispositif d'aspiration/refoulement 22, une fraction d'un échantillon 24 à analyser, sous forme liquide.

Afin de permettre le dépôt d'une ou plusieurs gouttes de l'échantillon 24 à l'intérieur de la cavité 58, il est procédé au décollement de la languette 62 (non représenté sur la figure 3B). Le décollement de la languette 62 peut être partiel ou total, comme explicité *supra.* Une fois la cavité 58 rendue accessible, le dispositif d'aspiration/refoulement 22, rempli avec la fraction d'échantillon 24 à analyser est positionnée en aplomb de la cavité 58, de façon à déposer au moins une goutte 64 de l'échantillon 24 au fond de ladite cavité 58. Il est à noter que le trou traversant 60 aménagé au fond de la cavité 58 doit présenter un diamètre suffisamment petit pour qu'il empêche le liquide constituée par la ou les gouttes 64 de se déverser à l'intérieur de la boite de Petri de manière passive. Mais, il doit également présenter un diamètre suffisamment grand pour permettre le transfert de l'échantillon liquide lorsqu'il est mis en oeuvre, en particulier par phénomènes vibratoires, tel que présenté ci-dessous.

Une fois la ou les gouttes 64 déposées, la languette 62 est alors repositionnée sur la cavité éliminant ainsi les risques de contamination. Ceci est présenté sur la figure 4C. Il est alors possible de générer des vibrations du couvercle 52 afin d'entraîner la pulvérisation de l'échantillon 24 à partir de la goutte 64, déposée dans la cavité 58, au travers du trou traversant 60. A cette fin, il est fait usage d'un moyen de vibration 28, mis en contact avec le couvercle 12, comme déjà explicité supra, en lien avec les figures 2C et 3C. Ces vibrations génèrent alors une pulvérisation par projection de microgouttes 30 d'échantillon à partir de la goutte 64, en particulier en direction du milieu de culture gélosé. Cette pulvérisation génère une répartition aléatoire à la surface du milieu de culture gélosé, de microgouttes d'échantillon susceptibles de contenir des microorganismes tels que des bactéries. Le résultat obtenu est ainsi sensiblement identique à un ensemencement traditionnel d'un milieu de culture par technique d'isolement.

Après incubation de la boite de Petri dans une étuve durant une période comprise entre 12 et 72h, on observe à la surface du milieu de culture gélosé 56, l'apparition de colonies bactériennes 32 isolées les unes des autres, tel que représenté sur la figure 4D.

## Revendications

1. Procédé d'ensemencement d'au moins un milieu de culture gélosé, contenu dans une boite de Petri, avec au moins un échantillon susceptible de contenir des microorganismes, ledit procédé comportant les étapes consistant à :
a. Fournir au moins un milieu de culture gélosé contenu dans une boite de Pétri ;
b. Fournir l'échantillon susceptible de contenir des microorganismes sous forme liquide ;
c. Effectuer au moins un dépôt de l'échantillon sous forme liquide sur le couvercle de la boite de Petri de sorte qu'un transfert dudit échantillon du couvercle vers le milieu de culture gélosé soit possible ;
d. Pulvériser l'échantillon, initialement déposé sur le couvercle, sur le milieu de culture gélosé, par mise en vibration de la boite de Petri.

2. Procédé selon la revendication 1, dans lequel le dépôt de l'échantillon est effectué sur la face intérieure du couvercle de la boite de Petri.

3. Procédé selon la revendication 2, dans lequel le dépôt de l'échantillon sur la face intérieure du couvercle, est effectuée au travers dudit couvercle.

4. Procédé selon la revendication précédente, dans lequel le dépôt de l'échantillon est réalisé au travers d'un septum.

5. Procédé selon la revendication 3, dans lequel le dépôt de l'échantillon est réalisé au travers d'un trou traversant réalisé dans le couvercle.

6. Procédé selon la revendication précédente, dans lequel le trou est réalisé par perçage avec l'outil de dépôt de l'échantillon.

7. Procédé selon la revendication 2, dans lequel la boite de Petri est ouverte afin d'effectuer le dépôt d'échantillon sur la face intérieur du couvercle de ladite boite de Petri.

8. Procédé selon la revendication 1, dans lequel le dépôt est effectué dans au moins une cavité, ménagée dans le couvercle.

9. Procédé selon la revendication précédente, dans lequel la cavité est ouverte sur la face extérieure du couvercle et présente un orifice au niveau de la face intérieur dudit couvercle.

10. Procédé selon l'une des revendications précédentes, dans lequel durant l'étape de pulvérisation, la vibration de la boite de Petri est réalisée par mise au contact de ladite boite avec un moyen de vibration.

11. Procédé selon la revendication précédente, dans lequel durant l'étape de vibration, le couvercle de la boite de Petri est mis au contact dudit moyen de vibration.

## Patentansprüche

1. Verfahren zum Einimpfen mindestens eines Agar-Kulturmediums, das in einer Petrischale enthalten ist, mit mindestens einer Probe, die Mikroorganismen enthalten könnte, wobei das Verfahren die folgenden Schritte umfasst:
a. Bereitstellen mindestens eines Agar-Kulturmediums, das in einer Petrischale enthalten ist;
b. Bereitstellen der flüssigen Probe, die Mikroorganismen enthalten könnte;
c. mindestens einmaliges Aufbringen der flüssigen Probe auf den Deckel der Petrischale, so dass ein Transfer der Probe vom Deckel zum Agar-Kulturmedium hin möglich ist;
d. Versprühen der Probe, die anfänglich auf den Deckel aufgebracht wurde, auf das Agar-Kulturmedium durch Inschwingungversetzen der Petrischale.

2. Verfahren nach Anspruch 1, wobei die Probe auf die Innenseite des Deckels der Petrischale aufgebracht wird.

3. Verfahren nach Anspruch 2, wobei die Probe durch den Deckel hindurch auf die Innenseite des Deckels aufgebracht wird.

4. Verfahren nach dem vorhergehenden Anspruch, wobei die Probe durch ein Septum hindurch aufgebracht wird.

5. Verfahren nach Anspruch 3, wobei die Probe durch ein im Deckel ausgeführtes Durchgangsloch hindurch aufgebracht wird.

6. Verfahren nach dem vorhergehenden Anspruch, wobei das Loch durch Bohren mit dem Werkzeug zum Aufbringen der Probe ausgeführt wird.

7. Verfahren nach Anspruch 2, wobei die Petrischale geöffnet wird, um die Probe auf die Innenseite des Deckels der Petrischale aufzubringen.

8. Verfahren nach Anspruch 1, wobei die Probe in mindestens einer im Deckel ausgebildeten Kavität aufgebracht wird.

9. Verfahren nach dem vorhergehenden Anspruch, wobei die Kavität auf der Außenseite des Deckels offen ist und eine Bohrung an der Innenseite des Deckels aufweist.

10. Verfahren nach einem der vorhergehenden Ansprüche, wobei während des Schritts des Versprühens das Schwingen der Petrischale durch Inkontaktbringen der Schale mit einem Schwingmittel ausgeführt wird.

11. Verfahren nach einem der vorhergehenden Ansprüche, wobei während des Schritts des Schwingens der Deckel der Petrischale mit einem Schwingmittel in Kontakt gebracht wird.

## Claims

1. A method for inoculating at least one agar culture medium, contained in a Petri dish, with at least one sample which may contain microorganisms, said method comprising the steps of:
a. providing at least one agar culture medium contained in a Petri dish;
b. providing the sample which may contain microorganisms in liquid form;
c. performing at least one deposition of the sample in liquid form onto the lid of the Petri dish such that said sample can be transferred from the lid to the agar culture medium;
d. spraying the sample, initially deposited onto the lid, onto the agar culture medium, by vibrating the Petri dish.

2. The method as claimed in claim 1, wherein the depositing of the sample is carried out onto the internal face of the lid of the Petri dish.

3. The method as claimed in claim 2, wherein the depositing of the sample onto the internal face of the lid, is carried out through said lid.

4. The method as claimed in the preceding claim, wherein the depositing of the sample is carried out through a septum.

5. The method as claimed in claim 3, wherein the depositing of the sample is carried out through a through-hole made in the lid.

6. The method as claimed in the preceding claim, wherein the hole is made by piercing with the tool for depositing the sample.

7. The method as claimed in claim 2, wherein the Petri dish is open in order to perform the sample deposition onto the internal face of the lid of said Petri dish.

8. The method as claimed in claim 1, wherein the deposition is carried out in at least one cavity, made in the lid,

9. The method as claimed in the preceding claim, wherein the cavity is open on the external face of the lid and has an orifice in the internal face of said lid.

10. The method as claimed in one of the preceding claims, wherein during the spraying step, the Petri dish is vibrated by bringing said dish into contact with a vibration means.

11. The method as claimed in the preceding claim, wherein, during the vibration step, the lid of the Petri dish is brought into contact with said vibration means.
